# EUROPÄISCHE PATENTANMELDUNG

(11) **EP 0 544 926 A1**
(43) Veröffentlichungstag der Anmeldung: **09.06.1993**
(21) Anmeldenummer: 91117405.0
(22) Anmeldetag: 11.10.1991
(51) Int. Cl.: A61L 2/08

(54) **Verfahren und Vorrichtung zur Sterilisation von Implantaten**

(71) Anmelder: Chiron Adatomed Pharmazeutische und Medizintechnische Gesellschaft mbH, D-85609 Dornach (DE)
(72) Erfinder: Lengfelder, Edmund, Prof.Dr.med., W-8000 München 82 (DE)
(74) Vertreter: Nöth, Heinz, Dipl.-Phys.

(57) **Zusammenfassung**

Ein Verfahren und eine Vorrichtung zur Sterilisation von Implantaten aus thermolabilen und strahlungsempfindlichen Stoffen, insbesondere Intraokularlinsen, bei denen die Sterilisationsbehandlung mit Alpha- oder Betastrahlen durchgeführt wird.

## Beschreibung

Die Erfindung betrifft ein Verfahren und eine Vorrichtung zur Sterilisation von Implantaten aus thermolabilen und strahlungsempfindlichen Stoffen, insbesondere von Intraokularlinsen.

Implantate, welche in den menschlichen Körper implantiert werden sollen, bestehen in vielen Fällen aus Thermoplasten, wie Polymethylmethacrylat, Polypropylen, Polyethylen und anderen Stoffen, welche weder durch Hitze noch durch Autoklavieren sterilisiert werden können. Das einzige derzeit einsetzbare Sterilisationsverfahren für thermolabile Gegenstände ist die Begasung mit Ethylenoxid. Allerdings ist der Einsatz von Ethylenoxid mit hohen Risiken verbunden, da dieses Sterilisationsmittel ein kanzerogener Stoff ist, ein hohes allgemeines Toxizitätspotential aufweist und bei der Implantation von Intraokularlinsen, die mit Ethylenoxid begast wurden, über das Auftreten intraokularer Reizzustände berichtet wurde. Ethylenoxid kann an einem damit sterilisierten Implantat schwere systemische Intoxikationen auslösen, wenn das im Biomaterial des Implantats sorbierte Gas sowie die durch Reaktion mit Wasser entstandenen Umsetzungsprodukte (Chlorhydrine) nicht quantitativ aus dem Biomaterial entfernt worden sind und stellt ferner aufgrund seiner hohen Toxizität eine starke Umweltbelastung dar. Trotz dieser Risiken wird Ethylenoxid routinemäßig zur Sterilisation von thermolabilen Arzneimitteln und thermolabilen Biomaterialien, wie Implantate, Nahtmaterialien, Operationshilfsmitteln etc. verwendet, da es derzeit die einzige sichere Methode darstellt, derartige thermolabile Stoffe zu sterilisieren.

Früher angewendete Verfahren, wie z. B. die Sterilisation mit 10 %iger Natronlauge (Naßsterilisation), erweisen sich als unzuverlässig. Bei der Sterilisation von Intraokularlinsen aus Polymethylmethacrylat (PMMA) sind in den USA 1978 zwei schwere epidemisch verlaufende intraokulare Infektionen durch pilzkontaminierte Natronlauge sowie durch pseudomonadenkontaminierte Neutralisationslösung aufgetreten. Aufgrund dieser Infektionsserie hat die amerikanische Gesundheitsbehörde FDA ab 1978 nur noch die Gassterilisation bei Intraokularlinsen zugelassen. Es besteht jedoch noch eine erhebliche Unsicherheit hinsichtlich der erlaubten Restgasmenge an Ethylenoxid im behandelten Gut. Die amerikanische Gesundheitsbehörde FDA erlaubt bei Intraokularlinsen einen Ethylenoxidrestgehalt sowie Chlorhydrinrestgehalt von jeweils 25 pmm pro Linse. Die französische Gesundheitsbehörde legt den maximalen Restgehalt an Ethylenoxid pro Linse mit 2 ppm fest. Das BGA empfiehlt einen Grenzwert von 1 ppm für Ethylenoxid und 150 ppm für chlorierte Ethylenhydrine bei Intraokularlinsen.

Beim alternativen Einsatz der Strahlensterilisation durch Gammabestrahlung ergibt sich die Gefahr der Strukturveränderung des Biomaterials, wodurch die Eignung des bestrahlten Implantats für den vorgesehenen medizinischen Zweck in Frage gestellt wird. Insbesondere dann, wenn das behandelte Biomaterial bei der Strahlenbehandlung vollständig von den Gammastrahlen durchdrungen wird, ergeben sich Materialänderungen, die die Eignung des Implantats dann in Frage stellen. Beispielsweise bei Intraokularlinsen haben die Linsen relativ dünne Fixationsfädchen, um die Linsen am Implantationsort im Körper fixieren zu können. Bei der Bestrahlung mit Gammastrahlen besteht die Gefahr, daß diese Fixationsfädchen brüchig werden und ihre Funktion dann nicht mehr erfüllen können. Außerdem führt Gammabestrahlung beim Linsenmaterial zu Verfärbungen und Trübungen. Wegen der hohen Risiken bei der Strahlensterilisation mit Gammastrahlen sind hohe Auflagen im Hinblick auf Qualitätsänderung, etwaige Radiolyseprodukte, Haltbarkeit und dgl. mehr zu erfüllen. Die für eine Zulassung der Strahlensterilisation erforderlichen Voraussetzungen sind daher derzeit bei im medizinischen Einsatz befindlichem Biomaterial nicht zu erbringen, so daß eine Substitution von Ethylenoxid durch Gammabestrahlung, und damit eine Strahlensterilisation, derzeit ausgeschlossen ist.

Aufgabe der Erfindung ist es daher, ein Verfahren und eine Vorrichtung zur Sterilisation von Implantaten aus thermolabilen und strahlungsempfindlichen Stoffen, insbesondere von Intraokularlinsen, zu schaffen, bei denen eine sichere Strahlensterilisation ohne wesentliche Änderung der Funktionalität des Implantatmaterials durchgeführt werden kann.

Diese Aufgabe wird beim eingangs genannten Verfahren erfindungsgemäß dadurch gelöst, daß die Sterilisationsbehandlung mit Alpha- oder Betastrahlen, die mit diffuser Strahlung auf die Implantatoberfläche gerichtet werden, durchgeführt wird. Es kann sich um bevorzugt eine diffuse oder auch gerichtete Strahlung handeln.

Bei der eingangs genannten Vorrichtung wird diese Aufgabe erfindungsgemäß dadurch gelöst, daß zwischen wenigstens zwei diffus strahlenden, als Alpha- oder Betastrahler ausgebildeten Strahlungsquellen ein Sterilisationsraum für zu sterilisierende Implantate vorgesehen ist.

Alphastrahlung von üblichen Radionukliden hat bei den in Frage kommenden Stoffen (mit spezifischem Gewicht von etwa 1) nur eine geringfügige Eindringtiefe bis zu einigen zehn Mikrometern, so daß die Gefahr von unkontrollierten Gefügeänderungen im für das Implantat verwendeten Biomaterial vermieden ist. Gleichzeitig wird jedoch durch die Alphastrahlung ein einwandfreie Sterilisation, d. h. Abtötung von lebenden und/oder entwicklungsfähigen Keimen erreicht. Auch bei Anwendung von Betastrahlung läßt sich bei entsprechender Bemessung der Elektronenenergie auf eine mittlere Elektronenenergie in der Größenordnung von einigen 10⁵ eV erreichen, daß die Betastrahlung nicht zu tief in das Biomaterial des Implantats eindringt, sondern nur an der Oberfläche des Implantats eine sterilisierende Wirkung hat.

Insbesondere bei der Anwendung der Erfindung bei Intraokularlinsen ist es von Bedeutung, daß das Linsenmaterial durch die Strahlungssterilisation nicht eingefärbt wird. Da weder die Alphastrahlung noch die anzuwendende Betastrahlung in das Linsenmaterial eindringt, besteht keinerlei Gefahr der Einfärbung. Auch ein Brüchigwerden des Materials ist vermieden.

In vorteilhafter Weise erreicht man daher bei der Erfindung eine Sterilisation, bei der das Zurückbleiben problematischer Giftstoffe ausscheidet und unerwünschte Materialveränderungen vermieden sind.

Bei Verwendung von Alphastrahlern werden die Implantate und die Umhüllungen, beispielsweise Einschweißfolien, getrennt im Vakuum sterilisiert. Zur Abtötung der Keime ist nur ein Bruchteil (ca. 5 bis 20 %) der Dosis erforderlich, wie sie bei der Betastrahlung zum Einsatz kommt. Allerdings ist es bei der Verwendung von Betastrahlen möglich, die zu sterilisierenden Implantate, beispielsweise Intraokularlinsen, schon eingeschweißt in den Einschweißfolien, die eine Dicke von etwa 0,05 mm aufweisen, zu bestrahlen und damit sowohl die Einschweißfolie als auch die darin eingeschlossene Intraokularlinse gleichzeitig zu sterilisieren. Ein Vakuum im Sterilisationsraum ist bei Betabestrahlung nicht erforderlich.

Sowohl bei der Alphabestrahlung als auch bei der Betastrahlung ist die an der Implantatoberfläche zur Wirkung kommende Dosis, an welcher die Keime sitzen, bedeutend höher als im bestrahlten Material, wodurch sich die gewünschte Wirkung der Sterilisation bei an der Oberfläche sitzenden Keimen erreichen läßt, ohne daß das Material selbst in seiner Qualität und in seinem Gefüge beeinträchtigt wird.

Als Strahlungsquellen eignen sich Radionuklide, beispielsweise als Alphastrahler Am-241 oder Am-243. Als Betastrahler eignet sich beispielsweise Tl-204 mit einer mittleren Energie der Gauß'schen Energieverteilung von etwa 240 keV. Diese Radionuklide können als Schichten auf eine ebene oder auch gewölbte Trägeroberfläche, beispielsweise eine Metallplatte, aufgebracht sein. Auch Pm-147 ist geeigneter Betastrahler. Insbesondere im Falle eines Betastrahlen emittierenden Nuklids, beispielsweise Sr-90/Y-90, kann über die Nuklidschicht noch eine Schutzfolie, z. B. aus Edelstahl mit einer Dicke von 0,02 mm, angebracht werden. Diese Folie hat in bevorzugter Weise auch die Funktion einer Streufolie, welche durch ihr Massenbremsvermögen die Elektronenenergie herabsetzt und dadurch die Eindringtiefe der Elektronen in das Implantat vermindert.

Die Strahlerfläche ist etwa der Fläche eines Einschweißbeutels oder sonstigen Hülle, in welcher die Intraokularlinse bzw. das Implantat sich befindet, angepaßt. Bevorzugt werden zwei Strahlerflächen verwendet, die im Abstand von etwa 1 cm einander gegenüberliegen. Es wird dadurch eine 4 pi-Geometrie erreicht. Die Einschweißbeutel mit den darin befindlichen zu sterilisierenden Gegenständen werden mit Fördervorrichtungen oder Metallvorrichtungen zwischen den Strahlern transportiert bzw. gehalten. Als gesamte Strahlenabschirmung genügt eine Blechverkleidung der Vorrichtung.

Das erfindungsgemäße Sterilisierungsverfahren eignet sich für alle Gegenstände, die keine Hohlkörper sind und deren Oberflächen von Keimen zu befreien sind.

In Abhängigkeit von der Art der jeweils abzutötenden Keime werden die Dosen eingestellt. Diese können im Bereich von einigen krad bis einigen hundert krad liegen. Je nach Höhe der Dosis kann dann die Verweilzeit des zu sterilisierenden Gegenstands im Sterilisationsraum eingestellt werden.

Anhand der beigefügten Figuren wird die Erfindung noch näher erläutert. Es zeigt:
- Fig. 1: ein erstes Ausführungsbeispiel der Erfindung;
- Fig. 2: ein zweites Ausführungsbeispiel der Erfindung; und
- Fig. 3: einen Teil des in Fig. 2 dargestellten Ausführungsbeispiels.

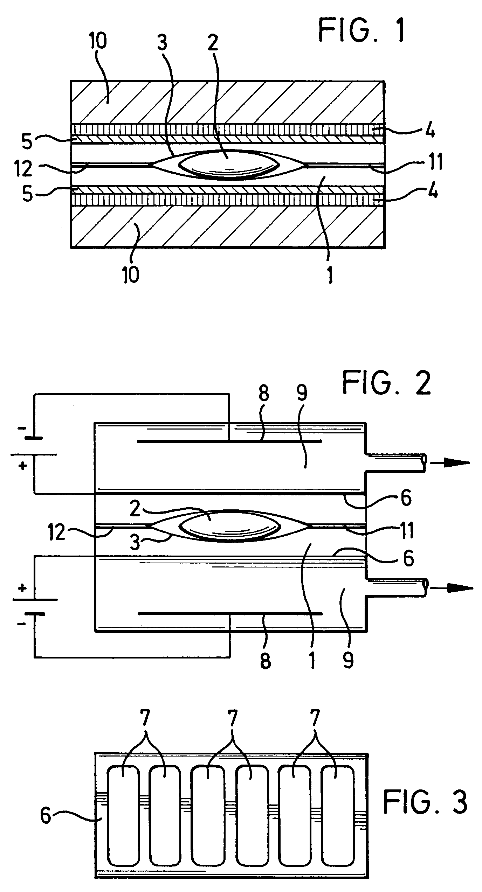

Bei dem in der Fig. 1 dargestellten Ausführungsbeispiel werden als radioaktive Teilchenstrahler Radionuklidschichten 4 verwendet. Diese können aus Am-241 oder Am-243 im Falle eines Alphastrahlers oder aus Sr-90/Y-90 im Falle eines Betastrahlers bestehen. Die Schichten können durch Aufdampfen oder auch durch Aufsintern auf eine Oberfläche eines Trägers 10 aufgebracht sein. Bei dem Träger 10 kann es sich um eine Metallplatte handeln. Insbesondere im Falle von Betastrahlen emittierenden Radionukliden ist über die Strahlerschicht 4 eine Schutzfolie 5, bevorzugt aus Edelstahl mit einer Dicke von etwa 0,02 mm, aufgebracht. Die beiden Strahler 4 liegen sich in einem Abstand von etwa 1 cm gegenüber und bilden zwischen sich einen Sterilisationsraum 1.

In diesem Sterilisationsraum 1 wird der zu sterilisierende Gegenstand, beispielsweise ein Implantat, das beim dargestellten Ausführungsbeispiel eine Intraokularlinse 2 ist, eingebracht. Diese Intraokularlinse 2 befindet sich in einer Einschweißhülle 3. Im Falle von Betastrahlern kann der zu sterilisierende Gegenstand in der Einschweißhülle 3 sterilisiert werden. Es werden dabei sowohl die Einschweißhülle 3 als auch der Implantatkörper, beispielsweie die Intraokularlinse 2, sterilisiert. In diesem Fall ist es nicht erforderlich, daß die Sterilisationskammer 1 evakuiert ist.

Falls Alphastrahler für die flächigen Strahlungsquellen 4 verwendet werden, wird die Sterilisationskammer 1 zur Erhöhung der Oberflächendosis evakuiert. Auch werden dann die Umhüllung 3 und der zu sterilisierende Gegenstand, beispielsweise die Intraokularlinse 2, getrennt sterilisiert.

In der Fig. 1 sind noch schematisch Haltevorrichtungen 11 und 12, an welchen die Einschweißhülle 3 und der eingepackte Gegenstand im Sterilisationsraum 1 gehalten werden, vorgesehen. Diese Halteeinrichtungen 11 und 12 können auch als Führungseinrichtungen beim Transport durch den Sterilisierungsraum 1 dienen. Beim Hindurchführen der Hülle 3 mit dem darin befindlichen Gegenstand 2 durch den Sterilisationsraum 1 ist die Transportgeschwindigkeit so eingestellt, daß die erforderliche Strahlungsdosis auf den eingepackten Gegenstand 2 sowie die Einschweißhülle 3 einwirkt. Es ist natürlich auch möglich, die Hülle 3 und den verpackten Gegenstand 2 stationär im Sterilisationsraum 1 während der Strahlungsbehandlung anzuordnen.

Bei dem in der Fig. 2 dargestellten Ausführungsbeispiel dient als Strahlungsquelle für Betastrahlen eine Glühkathode 8, die Betastrahlen bzw. Elektronen emittiert. Die Glühkathode 8 befindet sich in einer Vakuumkammer 9, die an eine nicht näher dargestellte Vakuumpumpe angeschlossen ist. Die Glühkathode 8 liegt an Hochspannung, damit die erforderlichen Elektronen (Glühemission) ausgesendet werden. Es kann sich hierbei um eine direkt beheizte Draht- oder Bandkathode aus hochschmelzendem Metall oder von indirekt durch einen gesonderten Heizdraht zum Glühen gebrachten Metallkörper, der beispielsweise mit Erdalkalioxiden belegt ist, handeln.

Der Kathode 8 gegenüberliegend befindet sich eine Fensterseite 6, durch welche die emittierten Elektronen in den Sterilisationsraum 1 eintreten und auf die Umhüllung 3 und den eingepackten Gegenstand 2, insbesondere die Intraokularlinse, einwirken. Auch hier können, wie beim Ausführungsbeispiel der Fig. 1, Halte- bzw. Transportvorrichtungen 11 und 12 für die Umhüllung 3 und den verpackten Gegenstand 2 vorgesehen sein.

Bei dem Ausführungsbeispiel in Fig. 3 ist die Fensterseite 6 so ausgebildet, daß sie streifenförmig ausgebildete elektronendurchlässige Fenster 7 aufweist (Fig. 3). Bei dieser Ausführungsform wird der zu sterilisierende Gegenstand mit einer Transportvorrichtung bewegt, so daß die zwischen den Fenstern 7 liegenden Stege und der dadurch bedingte "Strahlungsschatten" die Sterilisationsleistung nicht verringert.

Durch die in den Figuren dargestellten Ausführungsbeispiele wird eine Bestrahlung der im Sterilistionsraum eingebrachten Gegenstände an der Oberfläche der Gegenstände erreicht, ohne daß die Strahlung in das Material, insbesondere in das Material des verpackten Körpers, tiefer eindringt.

Bei den Ausführungsbeispielen sind zwei einander gegenüberliegende Strahlungsquellen mit flächiger Ausdehnung dargestellt. Es ist auch möglich, zwei weitere, senkrecht dazu sich erstreckende Strahlungsquellen vorzusehen, so daß der Sterilisationsraum 1 nicht nur oben und unten, sondern auch seitlich von Strahlungsquellen begrenzt ist, und nur an den Stirnseiten für den Transport und die Halterung der zu sterilisierenen Gegenstände zugänglich ist.

## Patentansprüche

1. Verfahren zur Sterilisation von Implantaten aus thermolabilen und strahlungsempfindlichen Stoffen, insbesondere von Intraokularlinsen, dadurch gekennzeichnet, daß die Sterilisationsbehandlung mit Alpha- oder Betastrahlen, die auf die Implantatoberfläche gerichtet werden, durchgeführt wird.

2. Verfahren nach Anspruch 1, dadurch gekennzeichnet, daß Betastrahlen mit einer mittleren Elektronenenergie von 0,5 bis einige 10⁵ eV) verwendet werden.

3. Verfahren nach Anspruch 1 oder 2, dadurch gekennzeichnet, daß als Strahlungsquellen Radionuklide verwendet werden.

4. Verfahren nach Anspruch 1 oder 2, dadurch gekennzeichnet, daß als Betastrahlungsquelle eine Glühkathode verwendet wird.

5. Vorrichtung zur Sterilisation von Implantaten aus thermolabilen und strahlungsempfindlichen Stoffen, insbesondere von Intraokularlinsen, zur Durchführung eines Verfahrens nach Anspruch 1, dadurch gekennzeichnet, daß zwischen wenigstens zwei als Alpha- oder Betastrahler ausgebildeten Strahlungsquellen (4; 6-9) ein Sterilisationsraum (1) für zu sterilisierende Implantate (2) angeordnet ist.

6. Vorrichtung nach Anspruch 5, dadurch gekennzeichnet, daß die Strahlungsquelle (4) eine auf einer Trägeroberfläche (10) aufgebrachte Radionuklidschicht ist und wenigstens zwei einander gegenüberliegende Radionuklidschichten den Sterilisationsraum (1) begrenzen.

7. Vorrichtung nach Anspruch 5 oder 6, dadurch gekennzeichnet, daß das Radionuklid Sr-90/Y-90, Tl-204 oder Pm-147 ist.

8. Vorrichtung nach Anspruch 5 oder 6, dadurch gekennzeichnet, daß das Radionuklid Am-241 oder Am-243 ist.

9. Vorrichtung nach einem der Ansprüche 6 bis 8, dadurch gekennzeichnet, daß zwischen dem Bestrahlungsgut und der Radionuklidschicht (4) eine Folie (5) angeordnet ist, welche die Funktion einer Schutzfolie und/oder einer Streufolie hat.

10. Vorrichtung nach Anspruch 5, dadurch gekennzeichnet, daß der Betastrahler als in einer Vakuumkammer (9) angeordnete elektronenemittierende Kathode (8) ausgebildet ist, und die Vakuumkammer (9) eine in den Sterilisationsraum (1) für die Elektronenstrahlen durchlässige Fensterseite (6) aufweist.

11. Vorrichtung nach Anspruch 10, dadurch gekennzeichnet, daß an der Fensterseite (6) mehrere streifenförmige, für die Elektronenstrahlen durchlässige Fenster (7) vorgesehen sind.
